# EUROPÄISCHE PATENTANMELDUNG

(11) **EP 0 786 266 A1**
(43) Veröffentlichungstag der Anmeldung: **30.07.1997**
(21) Anmeldenummer: 96120040.9
(22) Anmeldetag: 13.12.1996
(51) Int. Cl.: A61M 25/01

(54) **Katheterbesteck mit EKG-Ableitungsmöglichkeit**

(30) Priorität: 26.01.1996 DE 29601310 U
(71) Anmelder: B. BRAUN MELSUNGEN AG, 34212 Melsungen (DE)
(72) Erfinder: Wiegel, Heinz, Dipl.-Ing., 36211 Alheim (DE)
(74) Vertreter: Selting, Günther, Dipl.-Ing.

(57) **Zusammenfassung**

Das Katheterbesteck weist einen flexiblen Katheter (10) auf, der über einen Führungsdraht (17) in Seldinger-Technik verlegt wird. Zum Abgreifen von elektrischem Potential vom Führungsdraht (17) ist eine Klammer (18) vorgesehen, die seitlich an den Führungsdraht an beliebiger Stelle angesetzt werden kann. Damit kann das Potential unmittelbar hinter dem Katheteransatz (15) abgegriffen werden.

## Beschreibung

Die Erfindung betrifft ein Katheterbesteck mit EKG-Ableitungsmöglichkeit, mit einem flexiblen Katheter und einem elektrisch leitenden Führungsdraht, über den der Katheter schiebbar ist.

Aus EP-0 486 979 B1 ist ein Katheterbesteck mit EKG-Ableitungsmöglichkeit bekannt, bei dem der Katheter über einen in den Patientenkörper eingeführten Führungsdraht geschoben werden kann, bis die Katheterspitze das Zielgebiet erreicht hat. Der Führungsdraht wird mit einer Punktionskanüle in die zum rechten Vorhof des Herzens führende Hohlvene eingeschoben. Dabei ist an den Führungsdraht ein EKG-Gerät angeschlossen, mit dem die jeweilige Lage der Spitze des Führungsdrahtes ermittelt werden kann. Auf diese Weise kann ohne Röntgenüberwachung festgestellt werden, ob die Katheterspitze den Zielort erreicht hat. Bei einer anderen Vorgehensweise wird die EKG-Ableitung von dem Führungsdraht erst dann vorgenommen, wenn der Führungsdraht bis zum Zielort vorgeschoben und danach der Katheter spitzenbündig auf den Führungsdraht aufgeschoben worden ist. Sobald die EKG-Kurve die korrekte Verlegung der Katheterspitze anzeigt, wird der Führungsdraht aus dem Katheter herausgezogen. Für die EKG-Ableitung ist eine Klemmbuchse vorgesehen, die auf das proximale (patientenferne) Ende des Führungsdrahtes aufgesetzt wird und ein Anschlußteil für eine mit dem EKG-Gerät verbundene Steckvorrichtung aufweist. Hierbei ist der Verbinder somit am proximalen Ende des Führungsdrahtes angeordnet. Infolge des Gewichts des Verbinders und der daran befestigten Steckvorrichtung ist das Ende des Führungsdrahtes "kopflastig", wodurch der Führungsdraht dazu neigt, in den Katheter zurückgezogen zu werden und seine Lage zu verändern. Außerdem kann sich der am Katheterende befestigte Verbinder für die Handhabung des Katheterbestecks als nachteilig störend erweisen. Schließlich ist es auch erforderlich, den Verbinder nach dem Überschieben des Katheters über den Führungsdraht an dem Ende des Führungsdrahtes zu befestigen, das sich in einigem Abstand vom proximalen Katheterende befindet. Dabei muß der Arzt Manipulationen an verschiedenen Stellen des Katheterbestecks vornehmen.

Aus DE 43 19 033 C1 ist ferner ein Katheterbesteck bekannt, bei dem der Verbinder für den elektrischen Anschluß des Führungsdrahtes in einer Buchse angeordnet ist, die über den Führungsdraht geschoben und an dem Katheteransatz befestigt werden kann. Hierbei belastet der Verbinder zwar nicht den Führungsdraht, jedoch ist es erforderlich, die Buchse vom proximalen Ende her über eine erhebliche Länge des Führungsdrahtes zu schieben.

Der Erfindung liegt die Aufgabe zugrunde, ein Katheterbesteck mit EKG-Ableitungsmöglichkeit zu schaffen, das eine einfachere Anbringung des Verbinders am Führungsdraht ermöglicht.

Die Lösung dieser Aufgabe erfolgt erfindungsgemäß mit den im Anspruch 1 angegebenen Merkmalen.

Bei dem erfindungsgemäßen Katheterbesteck besteht der Verbinder aus einer seitlich an den Führungsdraht ansetzbaren Klammer. Dies hat den Vorteil, daß der Verbinder unmittelbar hinter dem Katheteransatz auf den Führungsdraht seitlich aufgesteckt werden kann und nicht vom proximalen Ende her über den Führungsdraht geschoben werden muß. Dabei kann der Arzt sich bei seinen Manipulationen auf den Bereich des Katheteransatzes konzentrieren. Sobald der Katheter spitzenbündig zum Führungsdraht vorgeschoben ist, kann die Klammer unmittelbar hinter dem Katheteransatz seitlich an dem Führungsdraht festgeklemmt werden. Damit wird zugleich ein unbeabsichtigtes Vorschieben des Führungsdrahts im Innern des Katheters verhindert.

Das erfindungsgemäße Katheterbesteck eignet sich insbesondere dazu, EKG-Ableitungen nach der Verlegung des Katheters vorzunehmen, wobei anhand der EKG-Kurve eine Feinpositionierung von Katheter und Führungsdraht vorgenommen werden kann. Generell kann die Klammer an allen Stellen des Führungsdrahtes angesetzt werden, wenn dieser blank ist. d.h. keine Isolierung aufweist. Es besteht auch die Möglichkeit, zunächst unter EKG-Überwachung einen isolierten Führungsdraht zu verlegen, anschließend die Klammer abzunehmen und dann den Katheter über den Führungsdraht zu schieben. Die Isolierung des isolierten Führungsdrahtes ist an einer oder mehreren vorgesehenen Kontaktstellen zur Anbringung der Klammer unterbrochen. Eine dritte Möglichkeit besteht darin, daß die Klammer einen Dorn aufweist, der beim Aufschieben der Klammer auf den Führungsdraht dessen Isolierung durchdringt und einen elektrischen Kontakt mit der Drahtseele herstellt.

Die Klammer kann zwei relativ zueinander manuell bewegbare Klemmteile aufweisen, die den Führungsdraht zwischen sich festklemmen, aus einer im wesentlichen starren elektrisch leitenden Schiebeklemme bestehen, die einen seitlich offenen Klemmspalt aufweist, oder aus zwei einstückig und elastisch miteinander verbundenen Klemmarmen bestehen, wobei ein Klemmarm eine elektrisch leitende Klemmbacke aufweist.

Bei einer bevorzugten Weiterbildung der Erfindung ist ein Halteteil vorgesehen, das die unmittelbar hinter dem Katheteransatz am Führungsdraht angreifende Klammer relativ zum Katheteransatz gegen axiales Verschieben sichert. Die Klammer greift mit dem Halteteil derart zusammen, daß sie relativ zu dem Katheteransatz nicht verschoben werden kann. Da die Klammer ihrerseits am Führungsdraht festgeklemmt wird, wird über das Halteteil eine axiale Lagesicherung des Führungsdrahtes in Bezug auf den Katheter erreicht. Dies bedeutet, daß der Führungsdraht im Katheter weder vor- noch zurückbewegt werden kann.

Im folgenden werden unter Bezugnahme auf die Zeichnungen Ausführungsbeispiele der Erfindung näher erläutert.

Es zeigen:
- Fig. 1: eine Darstellung des Katheterbestecks im verlegten Zustand während der EKG-Ableitung,
- Fig. 2: eine Seitenansicht der in Fig. 1 verwendeten Klammer wahrend des Klemmzustandes,
- Fig. 3: die Spitze der Klammer im Lösezustand,
- Fig. 4: die Schiebeklemme zur gegenseitigen Lagefixierung von Führungsdraht und Katheter,
- Fig. 5: einen am Katheteransatz befestigtes Halteteil, durch das die Klammer hindurchgreifen kann,
- Fig. 6: eine weitere Ausführungsform der Klammer,
- Fig. 7: eine dritte Ausführungsform der Klammer,
- Fig. 8: eine Klammer in Form einer elektrisch leitenden starren Schiebeklemme,
- Fig. 9: eine vierte Ausführungsform der Klammer mit aufspreizbaren Klemmschlitzschenkeln,
- Fig. 10: eine fünfte Ausführungsform der Klammer mit einem an dem Katheteransatz befestigten Halteteil, und
- Fig. 11: eine Draufsicht auf die Klammer der Fig. 10.

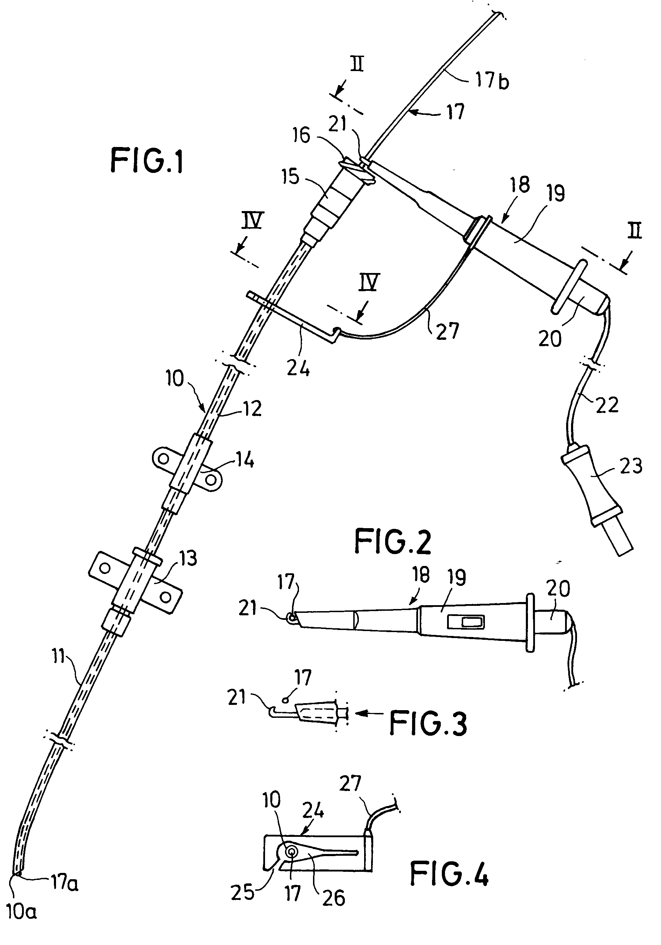

Das in Fig. 1 dargestellte Katheterbesteck weist einen Katheter 10 auf, der hier aus einem intrakorporalen ersten Katheterabschnitt 11 und einem extrakorporalen zweiten Katheterabschnitt 12 besteht. Der Katheterabschnitt 11 wird in den Patientenkörper eingeführt und er besteht aus einem körperverträglichen weichen Kunststoff. Der zweite Katheterabschnitt 12 bleibt außerhalb des Patientenkörpers. Er besteht aus durchsichtigem Kunststoff und ermöglicht den Einblick in das Katheterinnere zur Beobachtung des Führungsdrahtes und/oder von Flüssigkeit im Katheterinnern.

Auf dem ersten Katheterabschnitt 11 sitzt eine Fixiervorrichtung 13, die verschoben werden kann und dazu dient, das proximale Ende des Katheterabschnitts 11 an der Haut des Patienten zu befestigen. Eine weitere Fixiervorrichtung 14 ist am Übergang zwischen dem ersten Katheterabschnitt 11 und dem zweiten Katheterabschnitt 12 vorgesehen.

Am proximalen Ende des Katheters 10 befindet sich ein üblicher Katheteransatz 15, der am Ende des Katheterabschnitts 12 befestigt ist. Der Katheteransatz 15 weist einen Innenkonus auf und ist außen mit einem Lock-Verbinder 16 versehen, der das Anschließen einer Spritze oder einer Schlauchkupplung an den Katheter ermöglicht.

Zu dem Katheterbesteck gehört der Führungsdraht 17, der in den Katheter 10 eingeschoben werden kann. Im verlegten Zustand ist die Spitze 17a bündig mit der Katheterspitze 10a, während am proximalen Ende der rückwärtige Abschnitt 17b aus dem Katheteransatz 15 mit einer Länge von etwa 20 bis 30 cm herausragt. Der Führungsdraht 17 ist also wesentlich länger als der Katheter 10. Er weist eine Markierung auf, die bei Spitzengleichheit von Katheter und Führungsdraht am rückwärtigen Ende des Katheteransatzes gerade sichtbar ist.

Der Führungsdraht 17 besteht bei diesem Ausführungsbeispiel aus einem verseilten oder federförmig gewundenen blanken Draht, der eine gewisse Flexibilität hat und geradlinig ausgebildet ist. Der Spitzenabschnitt in der Nähe der Spitze 17a ist U-förmig umgebogen und von größerer Weichheit als der übrige Bereich, was in Fig. 1 jedoch nicht erkennbar ist, weil dort der Spitzenbereich durch den Katheter in gestrecktem Zustand gehalten wird.

Der Katheter 10 wird in Seldinger-Technik verlegt, indem die Vene zunächst mit einer (nicht dargestellten) Punktionskanüle punktiert und dann der Führungsdraht 17 durch die Punktionskanüle hindurch in die Vene bis ins Zielgebiet des Katheters vorgeschoben wird. Nach dem Zurückziehen der Punktionskanüle bleibt der Führungsdraht im Patientenkörper. Über den Führungsdraht 17 wird dann der Katheter geschoben, bis die Katheterspitze 10a bündig zur Spitze 17a des Führungsdrahtes 17 liegt. Die Spitzenbündigkeit kann anhand einer Markierung festgestellt werden, die am Führungsdraht 17 angebracht ist und die sich unmittelbar hinter dem Katheteransatz 15 befindet, wenn Spitzengleichheit hergestellt ist. Zur Durchführung der EKG-Ableitung wird die Klammer 18 seitlich an den Führungsdraht 17 angesetzt. Die Klammer 18 ist bei diesem Ausführungsbeispiel eine Potentialklemme, die einen langgestreckten Körper 19 aus Kunststoff aufweist, in dem ein aus dem rückwärtigen Ende herausragender federgespannter Schieber 20 angeordnet ist, welcher mit einem aus dem vorderen Ende vorschiebbaren Kontaktfinger 21 verbunden ist. Dieser Kontaktfinger 21 ist mit einem Kabel 22 verbunden, dessen Stecker 23 an das (nicht dargestellte) EKG-Gerät angeschlossen werden kann. Das Kabel 22 führt aus dem vorstehenden Ende des Schiebers 20 heraus. Zum Befestigen der Klammer 18 an dem Führungsdraht 17 wird der Halter 19 zwischen Zeigefinger und Mittelfinger festgehalten, während der Daumen axial gegen den federnd gespannten Schieber 20 drückt. Dadurch wird der hakenförmig gebogene Kontaktfinger 21 axial vorgeschoben (Fig. 3), so daß er den Führungsdraht 17 umgreifen kann. Wird der Schieber 20 losgelassen, so wird der Kontaktfinger 21 gegen den Körper 19 gezogen, wobei er gemäß Fig. 2 den Führungsdraht 17 festklemmt. Dabei entsteht ein sicherer Kontakt zwischen dem Kontaktfinger 21 und dem Führungsdraht 17. Die Klemmung ist so stark, daß Verschiebungen des Kontaktfingers 21 längs des Führungsdrahtes 17 verhindert werden.

Gemäß Fig. 1 ist ferner eine aus Kunststoff bestehende Schiebeklemme 24 vorgesehen, die in Fig. 4 dargestellt ist. Diese Schiebeklemme 24 weist einen seitlichen Einführschlitz 25 auf, der in eine keilförmige Klemmöffnung 26 hineinführt. Die Schiebeklemme 24 besteht aus elastischem Kunststoff. Sie wird seitlich über den Katheterabschnitt 12 mit dem darin liegenden Führungsdraht 17 geschoben, so daß der Katheter 10 in den Bereich der Keilöffnung 26 gelangt. Durch seitliches Verschieben der Schiebeklemme 24 wird der Katheter 10 in der Keilöffnung 26 festgeklemmt und gegen den Führungsdraht 17 gedrückt, wodurch der Führungsdraht im Katheter gegen axiales Verschieben gesichert wird. Die Schiebeklemme 24 ist mit einem Band 27 mit der Klammer 18 verbunden, so daß sie nicht verlorengehen oder herunterfallen kann.

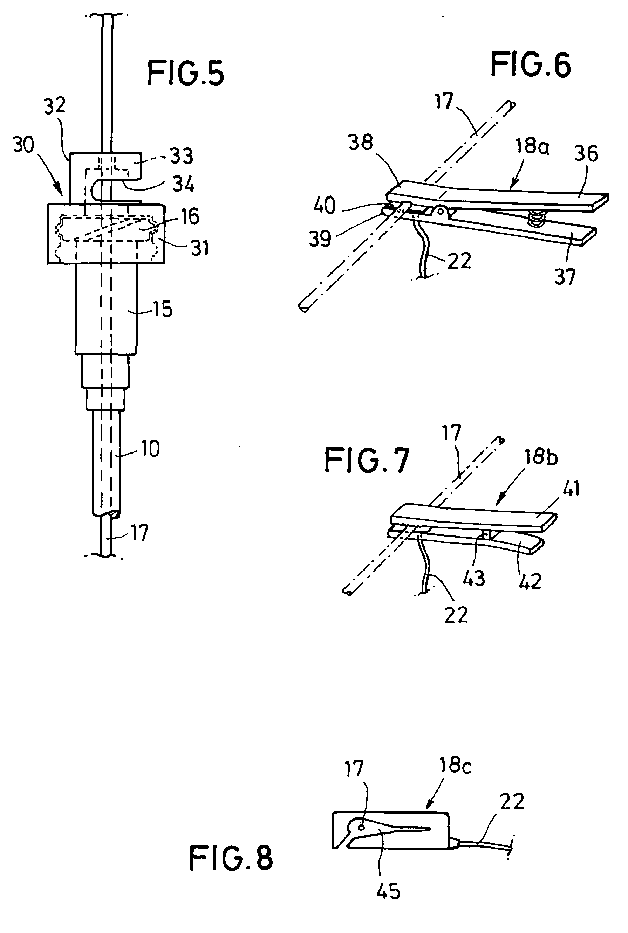

Bei dem Ausführungsbeispiel von Fig. 5 ist zusätzlich ein Halteteil 30 vorgesehen, das an dem Katheteransatz 15 befestigt werden kann. Diese Halteteil 30 besteht aus einer Lock-Mutter 31, die auf den Lock-Verbinder 16 aufgeschraubt werden kann, und einem axial davon abstehenden Ansatz 32, der einen seitlichen Schlitz 33 und eine radiale Ausnehmung 34 aufweist. Der Schlitz 33, der sich auch durch die Lock-Mutter 31, und somit über die gesamte Länge des Halteteils 30, erstreckt, dient dazu, das Halteteil seitlich über den Führungsdraht 17 schieben zu können, bevor das Halteteil auf den Lock-Verbinder 16 aufgeschraubt wird. Durch die Öffnung 34 hindurch wird die Klammer 18 an den Führungsdraht 17 angesetzt. Das Halteteil 30 macht die Schiebeklemme 24 entbehrlich. Es bewirkt eine Fixierung der an den Führungsdraht angesetzten Klammer 18 auch in proximaler Richtung, so daß über die Klammer der Führungsdraht 17 im Katheter 10 immobilisiert wird.

Fig. 6 zeigt eine andere Ausführungsform der Klammer 18a, die hier nach Art einer Wäscheklammer ausgebildet ist und zwei gelenkig miteinander verbundene Klammerschenkel 36,37 aufweist, die durch eine Feder auseinandergedrückt werden, wodurch Klemmbacken 38,39, die den Führungsdraht 17 umschließen, gegeneinandergedrückt werden. Eine der Klemmbacken ist mit einem Kontaktstück 40 versehen, welches mit dem zum EKG-Gerät führenden Kabel 22 verbunden ist.

Eine ähnliche Klammer 18b ist in Fig. 7 dargestellt. Hierbei sind die Klammerschenkel 41,42 mit einem elastischen Biegesteg 43 aus Kunststoff einstückig ausgebildet, der bewirkt, daß die Klemmbacken gegeneinandergedrückt werden. Durch Zusammendrücken der Klammerschenkel kann die Klammer geöffnet werden.

Fig. 8 zeigt eine Klammer 18c in Form einer einstückigen Schiebeklemme, die einen seitlich offenen Klemmspalt 45 aufweist und aus elektrisch leitendem Material besteht. Die Schiebeklemme hat eine ähnliche Form wie die Schiebeklemme 24 der Fign. 1 und 4. Sie dient jedoch ausschließlich zum Einklemmen des Führungsdrahtes 17, den sie auf diese Weise elektrisch mit dem Kabel 22 verbindet.

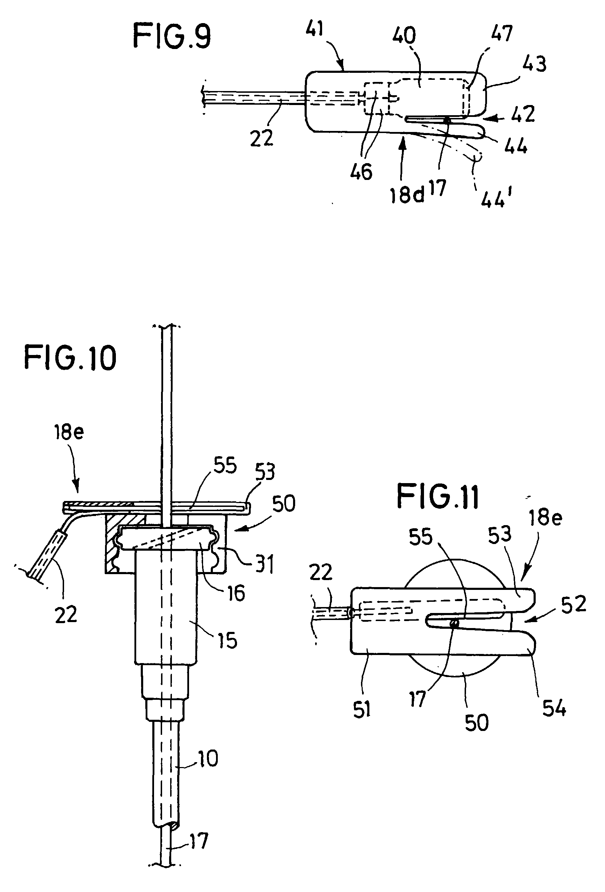

Eine weitere Ausführungsform einer Klammer 18d zeigt Fig. 9. Die Klammer 18d besteht im wesentlichen aus einem Kontaktblech 40, an dem das Kabel 22 befestigt ist und das in einem Gehäuseblock 41 aus nicht leitendem Kunststoff eingegossen ist. Der einstückige Gehäuseblock 41 weist einen Schlitz 42 auf, der zwei Klemmarme 43,44 des Gehäuseblocks 41 trennt. Die beiden Klemmarme 43,44 sind über den Gehäusekörper elastisch miteinander verbunden. Der schmalere Klemmarm 44 kann von dem breiteren Klemmarm 43 in eine abgespreizte Position 44' weggebogen werden, so daß der Schlitz 42 breiter wird und eine V-förmige Form annimmt.

Das Kontaktblech 40 ist so in den Gehäuseblock 41 eingegossen, daß eine Längskante des Kontaktblechs 40 aus dem größeren Klemmarm 43 zur Schlitzinnenseite aus dem Klemmarm 43 stegartig herausragt.

An dem dem Schlitz 42 abgewandten Ende des Gehäuseblocks 41 ist die Drahtseele des Kabels 22 mit zwei Klemmzungen 46 des Kontaktblechs 40 mit dem Kontaktblech 40 elektrisch und mechanisch verbunden.

Die Klammer 18d wird auf den Führungsdraht 17 aufgesteckt, indem der schmalere Klemmarm 44 manuell von dem breiteren Klemmarm 43 abgespreizt wird. In diesem Zustand wird die Klammer 18d mit dem V-förmigen geöffneten Schlitz 42 auf den Führungsdraht 17 gesteckt. Wenn der schmalere Klemmarm 44 schließlich wieder losgelassen wird, klemmt er den Führungsdraht 17 in dem nun wieder engen und schmalen Schlitz 42 fest. Dabei wird der Führungsdraht 17 an das Kontaktblech 40 gedrückt, wodurch ein sicherer elektrischer Kontakt zwischen dem Führungsdraht 17 und dem Kontaktblech 40 gewährleistet ist. Die Klammer 18d dieser Ausgestaltung ist an dem Führungsschaft 17 montierbar, ohne daß dieser dabei verschoben oder verdreht wird.

Das vordere Ende 47 des Kontaktblechs 40 ist zu allen Seiten hin abgeschrägt. Die Abschrägung an der dem Schlitz 42 zugewandten Seite bewirkt, daß beim Aufstecken der Klammer 18d das Kontaktblech 40 nicht an dem Führungsdraht 17 hängenbleiben kann, sondern der Führungsdraht 17 an der Abschrägung entlang in den Schlitz 42 geführt wird.

Die Fign. 10 und 11 zeigen eine weitere Ausführungsform, bei der eine Klammer 18e Bestandteil des Halteteils 50 ist, das auf den in Fig. 5 gezeigten Lock-Verbinder 16 aufgeschraubt wird. An eine ebenfalls schon in Fig. 5 gezeigte geschlitzte Lock-Mutter 50 ist das Klemmteil 51 angeklebt. Das Klemmteil 51 weist einen Schlitz 52 auf, der zwei gleiche Klemmarme 53,54 trennt und der mit dem Schlitz 31 der Lock-Mutter 50 ausgerichtet ist. In einen Klemmarm 54 ist ein Kontaktblech 55 einlassen, das zur Schlitzinnenseite aus dem Klemmarm 54 herausragt. Mit dem Kontaktblech 55 ist das Kabel 22 durch Verlötung elektrisch und mechanisch verbunden.

Zum Abgreifen elektrischer Signale von dem Führungsdraht 17 wird die Klammer 18e seitlich über den Führungsdraht 17 geschoben. Dabei wird der Führungsdraht 17 in die Schlitze 31,52 der Lock-Mutter 50 und des Klemmteils 51 geschoben, woraufhin die Lock-Mutter 50 auf den Lock-Verbinder 16 aufgeschraubt wird. Dabei wird der Führungsdraht 17 weiter in den Schlitz 52 hineingedreht, so daß der Führungsdraht 17 fest gegen das Kontaktblech 55 gedrückt wird, und ein guter elektrischer Kontakt zwischen dem Führungsdraht 17, dem Kontaktblech 55 und dem Kabel 22 bewirkt wird.

## Patentansprüche

1. Katheterbesteck mit EKG-Ableitungsmöglichkeit, mit einem flexiblen Katheter (10), der an seinem proximalen Ende einen Katheteransatz (15) aufweist, einem Führungsdraht (17), über den der Katheter schiebbar ist, und einem an dem Führungsdraht (17) anbringbaren Verbinder zum Abgreifen von elektrischem Potential des Führungsdrahtes (17),
**dadurch gekennzeichnet,**
daß der Verbinder aus einer seitlich an den Führungsdraht (17) ansetzbaren Klammer (18) besteht.

2. Katheterbesteck nach Anspruch 1, dadurch gekennzeichnet, daß die Klammer (18) zwei relativ zueinander manuell bewegbare Klemmteile aufweist, die den Führungsdraht (17) zwischen sich festklemmen.

3. Katheterbesteck nach Anspruch 1, dadurch gekennzeichnet, daß die Klammer (18c) aus einer im wesentlichen starren elektrisch leitenden Schiebeklemme besteht, die einen seitlich offenen Klemmspalt (45) aufweist.

4. Katheterbesteck nach Anspruch 1, dadurch gekennzeichnet, daß die Klammer (18d) aus zwei einstückig miteinander ausgebildeten Klemmarmen (43,44) besteht, die elastisch miteinander verbunden sind, wobei einer der Klemmarme (43) eine elektrisch leitende Klemmbacke (40) aufweist.

5. Katheterbesteck nach einem der Ansprüche 1 bis 4, dadurch gekennzeichnet, daß eine Schiebeklemme (24) vorgesehen ist, die den Katheter (10) und den darin befindlichen Führungsdraht (17) relativ zueinander fixiert, und daß die Schiebeklemme (24) unverlierbar mit der Klammer (18) verbunden ist.

6. Katheterbesteck nach einem der Ansprüche 1 bis 5, dadurch gekennzeichnet, daß ein Halteteil (30) vorgesehen ist, das die unmittelbar hinter dem Katheteransatz (15) am Führungsdraht (17) angreifende Klammer (18) relativ zum Katheteransatz (15) gegen axiales Verschieben sichert.

7. Katheterbesteck nach Anspruch 6, dadurch gekennzeichnet, daß das Halteteil (30) eine auf den Katheteransatz (15) schraubbare Lock-Mutter (31) aufweist, die mit einem längslaufenden Schlitz zum seitlichen Überschieben über den Führungsdraht (17) versehen ist.

8. Katheterbesteck nach Anspruch 6 oder 7, dadurch gekennzeichnet, daß die Klammer (18e) Bestandteil des Halteteils (50) ist.
